# EUROPEAN PATENT APPLICATION

(11) **EP 4 574 882 A1**
(43) Date of publication of application: **25.06.2025**
(21) Application number: 23855080.0
(22) Date of filing: 02.08.2023
(51) Int. Cl.: C08G 63/60, C08G 63/181, C08G 63/78, C07C 67/31

(54) **METHOD FOR PREPARING BIS(GLYCOL)TEREPHTHALATE OLIGOMER AND POLYESTER RESIN**

(30) Priority: 18.08.2022 KR 20220103171
(71) Applicant: SK Chemicals Co., Ltd., Seongnam-si, Gyeonggi-do 13494 (KR)
(72) Inventor: HWANG, Da-Young, Seongnam-si, Gyeonggi-do 13494 (KR); LEE, Yoo Jin, Seongnam-si, Gyeonggi-do 13494 (KR)
(74) Representative: Haseltine Lake Kempner LLP
(86) International application number: PCT/KR2023/011294
(87) International publication number: WO 2024/039113

(57) **Abstract**

According to the present invention, bis(glycol)terephthalate can be prepared by a transesterification reaction of glycol and bis(2-hydroxyethyl)terephthalate and then introduced to a polymerization reaction to increase the content of recycled raw materials in a polyester resin. In addition, purity is improved through the removal of impurities by extracting glycol byproducts during the transesterification reaction, and thus the quality of the final resin can be improved while reducing the amount of byproducts from the polymerization reaction.

## Description

### Technical Field

The present invention relates to a recycled bis(glycol) terephthalate oligomer and to a process for preparing a polyester resin using the same.

### Background Art

Polyester is widely used as a material for beverage-filling containers, packaging films, audio and video films, and the like by virtue of its excellent mechanical strength, thermal resistance, transparency, and gas barrier properties. In addition, polyester is widely produced worldwide as an industrial material such as medical fibers and tire cords. In particular, polyester sheets or plates have good transparency and excellent mechanical strength, so that they are widely used as raw materials for cases, boxes, partitions, shelves, panels, packaging materials, building materials, interior and exterior materials, and the like.

As a result, waste of plastics such as polyester is generated globally at an unmanageable level every year. Recently, countries around the world have prepared regulations and plans for recycling waste plastic resources, including waste polyester. For example, there is an attempt to use a recycled resin in packaging materials used in various fields at a certain ratio or more. Although physical or chemical methods are used as methods of recycling waste polyester, physical recycling methods cannot guarantee purity and are not widely used.

In chemical recycling methods, the ester bond of waste polyester is cleaved to depolymerize it. Reactions such as glycolysis, hydrolysis, methanolysis, and aminolysis are used. Glycolysis among them is to decompose waste polyester by adding a glycol such as ethylene glycol or diethylene glycol at high temperatures. A reaction product containing mainly bis(2-hydroxyethyl) terephthalate (BHET) is obtained. The bis(2-hydroxyethyl) terephthalate may be used as a raw material for preparing unsaturated polyester or ester polyol after the crystallization or purification thereof.

However, from the viewpoint of environmental friendliness, it is more meaningful to convert waste PET products, which generally have a short lifespan, into engineering polyester products or environmentally friendly biodegradable polyester products that have a long lifespan. In this regard, recently, attempts have been made to regenerate polyester resins other than PET through the transesterification of waste PET and a glycol. Since an excessive amount of a glycol is used in this reaction and the reaction time is long, there is a problem in that a large amount of by-products such as cyclic ester compounds are formed as a side reaction of the glycol.

### [Prior Art Document]

(Non-patent Document 1) Park, S.H., Kim, S.H., Poly(ethylene terephthalate) recycling for high value added textiles, Fashion and Textiles 1, 1 (2014)

### Disclosure of the Invention

### Technical Problem

The present inventors have attempted to develop a method capable of enhancing the quality and economic efficiency of a final product while increasing the content of recycled raw materials through a technology to recycle BHET obtained by the depolymerization of waste PET-based products into various engineering polyester products or environmentally friendly biodegradable polyester products.

Meanwhile, recycled BHET has generally low purity due to impurities formed from the reagents used in the depolymerization process and side reactions, so that separate processes such as ion exchange or recrystallization are required to purify it, thereby causing a problem of increased costs. In addition, glycol monomers (DEG, ISB, CHDM, and the like) must be used in addition to BHET in order to produce a copolyester resin, making it difficult to increase the content of recycled raw materials in the final resin. When an excessive amount of BHET is added to the polymerization reaction to increase the content of recycled raw materials in the conventional process, there is a problem of increased glycol by-products.

As a result of research conducted by the present inventors, it has been discovered that, when a glycol for copolymerization is subjected to a transesterification reaction with BHET in advance to prepare a bis(glycol) terephthalate, which is then subjected to a polymerization reaction, the content of recycled raw materials in the polyester resin can be increased; and purity is improved by collecting glycol by-products and removing impurities during the transesterification reaction, thereby enhancing the quality of the final resin, along with a decrease in by-products during the polymerization reaction.

Accordingly, an object of the present invention is to provide a process for preparing a polyester resin with a high content of recycled raw materials such as BHET, a decrease in by-products of the polymerization reaction, and excellent quality of the final resin, and a polyester resin prepared thereby.

### Solution to the Problem

According to the present invention, there is provided a process for preparing a polyester resin, which comprises subjecting bis(2-hydroxyethyl) terephthalate to a transesterification reaction with a glycol having 3 or more carbon atoms; collecting and removing ethylene glycol as a by-product formed during the transesterification reaction; obtaining a bis(glycol) terephthalate oligomer as a product of the transesterification reaction; and conducting a polymerization using the bis(glycol) terephthalate oligomer.

According to the present invention, there is provided a process for preparing a bis(glycol) terephthalate oligomer, which comprises subjecting bis(2-hydroxyethyl) terephthalate to a transesterification reaction with a glycol having 3 or more carbon atoms; and collecting and removing ethylene glycol as a by-product formed during the transesterification reaction.

In addition, according to the subject invention, there are provided a polyester resin and a bis(glycol) terephthalate oligomer prepared by the above processes.

### Advantageous Effects of the Invention

According to the present invention, bis(2-hydroxyethyl) terephthalate as a recycled raw material obtained by the depolymerization of waste polyester is subjected to a transesterification reaction with a glycol suitable for the desired copolymerization composition in advance to prepare a bis(glycol) terephthalate oligomer, which is then subjected to a polymerization reaction. As a result, the content of recycled raw materials can be increased even without adding an excessive amount of bis(2-hydroxyethyl) terephthalate to the polymerization reaction of a polyester resin.

In particular, according to the present invention, as the glycol by-product (ethylene glycol) is collected and removed during the transesterification reaction, the glycol by-product formed in the subsequent polymerization reaction of a polyester resin can be reduced, and the recovered glycol can be reused in the polymerization reaction, whereby the costs for raw materials and process can also be reduced. The purity can be improved by removing impurities, resulting in a final resin with enhanced quality, which can be applied to the preparation of environmentally friendly polyester products.

### Brief Description of the Drawing

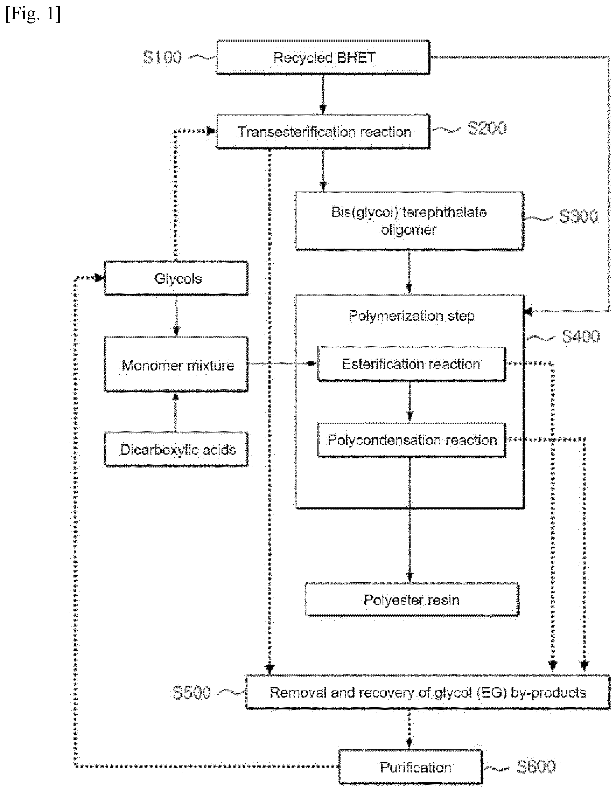

Fig. 1 schematically depicts a process for preparing a polyester resin according to an embodiment of the present invention.

### Best Mode for Carrying out the Invention

In this specification, terms referring to the respective components are used to distinguish them from each other and are not intended to limit the scope of the embodiment. In addition, in the present specification, a singular expression is interpreted to cover a plural number as well unless otherwise specified in the context.

In the present specification, the terms first, second, and the like are used to describe various components. But the components should not be limited by the terms. The terms are used for the purpose of distinguishing one element from another.

In the present specification, the term "comprising" is intended to specify a particular characteristic, region, step, process, element, and/or component. It does not exclude the presence or addition of any other characteristic, region, step, process, element, and/or component, unless specifically stated to the contrary.

The molecular weight of a compound or polymer described in the present specification, for example, a number average molecular weight or a weight average molecular weight, is a relative mass based on carbon-12 as is well known. Although its unit is not described, it may be understood as a molar mass (g/mole) of the same numerical value, if necessary.

In the present specification, a "derivative" of a specific compound refers to a compound obtained by partially transforming the compound through a chemical reaction or combining it with other components, thereby containing the main part of the compound.

In the present specification, a unit or group "derived" from a specific component refers to a part of the component contained in a final product through a chemical reaction such as a polymerization reaction. It may be present in a form in which a part thereof is modified during the reaction or it is combined with other components. For example, a unit or group derived from at least one monomer or oligomer is contained in the chain constituting a polymer.

In the present specification, a recycled monomer or oligomer may refer to a monomer or oligomer obtained by decomposing, depolymerizing, reprocessing, or repolymerizing waste plastics such as waste polyester by physical or chemical methods, or polymerization raw materials containing the same or derived from the same.

As an example, bis(2-hydroxyethyl) terephthalate (BHET) used as a starting material in the process according to the present invention may be a recycled monomer obtained by the depolymerization of waste polyester.

As another example, recycled monomers may also be used as a glycol having 3 or more carbon atoms subjected to a transesterification reaction in the process of the present invention. As a specific example, at least one type of recycled glycol, such as recycled 1,4-cyclohexanedimethanol (r-CHDM), recycled diethylene glycol (r-DEG), and recycled isosorbide (r-ISB) may be used.

As another example, recycled monomers may also be used as a comonomer subjected to a polymerization reaction for a polyester resin in the process of the present invention. As a specific example, at least one type selected from recycled dicarboxylic acids and recycled diols may be used. As a more specific example, at least one type of recycled monomer, such as recycled ethylene glycol (r-EG), recycled 1,4-cyclohexanedimethanol (r-CHDM), recycled diethylene glycol (r-DEG), recycled isosorbide (r-ISB), recycled terephthalic acid (r-TPA), recycled isophthalic acid (r-IPA), and recycled dimethyl terephthalate (r-DMT) may be used.

Such recycled monomers may be obtained directly from waste plastics such as waste polyester by known methods or can be purchased commercially and used.

In addition, the bis(glycol) terephthalate oligomer (e.g., BHDT oligomer, BHIT oligomer, BHCT oligomer, or the like) obtained as a product of a transesterification reaction according to the present invention may be a recycled oligomer obtained by decomposing, depolymerizing, reprocessing, or repolymerizing waste plastic, such as waste polyester, by physical or chemical methods.

Such recycled raw materials such as recycled monomers or oligomers can be used in the preparation of a polyester resin in the present invention.

The present invention provides a process for preparing a polyester resin, which comprises subjecting bis(2-hydroxyethyl) terephthalate to a transesterification reaction with a glycol having 3 or more carbon atoms; collecting and removing ethylene glycol as a by-product formed during the transesterification reaction; obtaining a bis(glycol) terephthalate oligomer as a product of the transesterification reaction; and conducting a polymerization using the bis(glycol) terephthalate oligomer.

Fig. 1 schematically depicts a process for preparing a polyester resin according to an embodiment of the present invention. Referring to Fig. 1, the process for preparing a polyester resin according to an embodiment comprises preparing recycled bis(2-hydroxyethyl) terephthalate obtained by the depolymerization of waste polyester (S100); subjecting it to a transesterification reaction with a glycol having 3 or more carbon atoms (S200); collecting and removing ethylene glycol as a by-product (S500); and obtaining a bis(glycol) terephthalate oligomer (S300). For a subsequent polymerization, bis(2-hydroxyethyl) terephthalate, a bis(glycol) terephthalate oligomer, and other monomer mixtures are added to an esterification reaction, followed by a polycondensation reaction (S400), to obtain a polyester resin. Glycol by-products may also be collected and removed during the esterification reaction and/or polycondensation reaction (S500). The glycol thus recovered may be purified (S600) and then supplied again to the transesterification reaction and/or polymerization step.

According to the present invention, bis(2-hydroxyethyl) terephthalate as a recycled raw material obtained by the depolymerization of waste polyester is subjected to a transesterification reaction with a glycol suitable for the desired copolymerization composition in advance to prepare a bis(glycol) terephthalate oligomer, which is then subjected to a polymerization reaction. As a result, the content of recycled raw materials can be increased even without adding an excessive amount of bis(2-hydroxyethyl) terephthalate to the polymerization reaction of a polyester resin.

In particular, according to the present invention, as the glycol by-product (ethylene glycol) is collected and removed during the transesterification reaction, the glycol by-product formed in the subsequent polymerization reaction of a polyester resin can be reduced, and the recovered glycol can be reused in the polymerization reaction, whereby the costs for raw materials and process can also be reduced. The purity can be improved by removing impurities, resulting in a final resin with enhanced quality, which can be applied to the preparation of environmentally friendly polyester products.

Hereinafter, the present invention will be described in more detail.

### Bis(2-hydroxyethyl) terephthalate

Bis(2-hydroxyethyl) terephthalate is an ester of two ethylene glycols and one terephthalic acid. For example, it is a compound formed as an intermediate in the process of preparing a polyester such as polyethylene terephthalate (PET) through the polymerization of ethylene glycol and terephthalic acid or its ester.

The bis(2-hydroxyethyl) terephthalate used in the present invention may be obtained by the depolymerization of waste polyester. For example, the bis(2-hydroxyethyl) terephthalate may be obtained from waste polyester having a repeat unit of ethylene glycol and terephthalic acid like polyethylene terephthalate (PET) or glycol-modified polyethylene terephthalate (PETG). Specifically, it may be obtained by well-known depolymerization methods such as glycolysis, hydrolysis, and methanolysis. In particular, the bis(2-hydroxyethyl) terephthalate may be obtained by depolymerizing waste polyethylene terephthalate with ethylene glycol and then purifying it.

Bis(2-hydroxyethyl) terephthalate obtained by the depolymerization of waste polyester as described above is referred to as "recycled bis(2-hydroxyethyl) terephthalate (recycled BHET)," or abbreviated as r-BHET or rBHET, which needs to be understood as distinct from a pure BHET compound. Specifically, recycled BHET may contain reagents or solvents used in various chemical steps during the depolymerization of waste polyester or by-products formed by side reactions with them. Thus, BHET recycled by a common depolymerization process contains organic and inorganic impurities in addition to BHET as the main component; thus, its purity is not high. For this reason, recycled BHET can also be viewed as a kind of composition comprising two or more components, i.e., a BHET composition. Such recycled BHET may be used as a polymerization raw material for producing a polyester resin.

Impurities contained in the recycled BHET may comprise, for example, diethylene glycol derivatives and unreacted monomers. The total content of impurities contained in the recycled BHET may be 10% by weight or more, 15% by weight or more, or 20% by weight or more, and may be 40% by weight or less, 35% by weight or less, 30% by weight or less, or 25% by weight or less.

The purity of the recycled BHET may be measured using liquid chromatography or the like. Specifically, the purity of the recycled BHET may be calculated by measuring the fraction (%) of the peak area of BHET out of the total peak area in a spectrum obtained using high-performance liquid chromatography (HPLC).

For example, the purity of the recycled BHET may be 97% or less, 90% or less, 85% or less, or 80% or less, and may be 60% or more, 65% or more, or 70% or more. Specifically, the purity of the BHET introduced into the transesterification reaction of the present invention may be 60% to 97%, more specifically, 65% to 90%, 65% to 85%, or 70% to 80%.

### Glycol

The glycol used in the transesterification reaction of the present invention is employed in a transesterification reaction with bis(2-hydroxyethyl) terephthalate to form a residue in a bis(glycol) terephthalate oligomer as a product. It constitutes the polymer chain of a final polyester resin polymerized therefrom.

The glycol used in the present invention may be a glycol having 3 or more carbon atoms for substituting an ethylene glycol residue in a transesterification reaction.

In particular, the glycol has a boiling point higher than that of ethylene glycol by 10°C or more, which is advantageous for purification through fractional distillation during the transesterification reaction.

Specifically, the glycol used in the transesterification reaction of the present invention may be a glycol monomer other than ethylene glycol (e.g., alkylene glycol having 3 or more carbon atoms) or a polymer glycol (e.g., polyether).

As a more specific example, the glycol may be at least one selected from glycol monomers having 3 to 20 carbon atoms and having a molecular weight of less than 500 and polymeric glycols having a number average molecular weight of 400 to 5,000.

The number of carbon atoms of the glycol monomer may be, for example, 3 or more or 4 or more, and may be 20 or less, 15 or less, 12 or less, 10 or less, or 8 or less.

Specifically, the glycol monomer may be an aliphatic diol having 3 to 20 carbon atoms. In addition, the aliphatic diol may be a chain type or a cyclic type.

As a specific example, the glycol having 3 or more carbon atoms may comprise at least one selected from the group consisting of 1,3-propanediol, 1,4-butanediol, 1,4-cyclohexanedimethanol, isosorbide, 2-methyl-1,3-propanediol, 2-methylene-1,3-propanediol, 2-ethyl-1,3-propanediol, 2-isopropyl-1,3-propanediol, 2,2-dimethyl-1,3-propanediol, 2,3-butanediol, 3-methyl-1,5-pentanediol, 3-methyl-2,4-pentanediol, 1,6-hexanediol, 1,2-cyclohexanediol, 1,4-cyclohexanediol, diethylene glycol, polyethylene glycol, polypropylene glycol, polytetramethylene glycol, polyhexamethylene glycol, copolymers of ethylene oxide and tetrahydrofuran, ethylene oxide-adducted polypropylene glycol, polycarbonate diol, polyneopentyl glycol, poly-3-methylpentanediol, poly-1,5-pentanediol, and derivatives thereof.

As a more specific example, the glycol monomer may comprise at least one selected from the group consisting of diethylene glycol, isosorbide, 1,4-cyclohexanedimethanol, and derivatives thereof. Such a glycol monomer may be a virgin glycol.

As another example, the glycol having 3 or more carbon atoms may comprise at least one type of recycled glycol obtained by the depolymerization of waste polyester. As a specific example, the recycled glycol may be selected from the group consisting of recycled 1,4-cyclohexanedimethanol, recycled diethylene glycol, and recycled isosorbide. However, the type of recycled glycol that can be used in the present invention is not limited thereto. Any recycled glycol that can be subjected to a transesterification reaction can be used. Such a recycled glycol may be used in the transesterification reaction to completely replace a virgin glycol. Alternatively, a recycled glycol and a virgin glycol may be mixed at a certain ratio and used in the transesterification reaction. The mixing ratio of the recycled glycol may be, for example, 0% by mole or more, 1% by mole or more, 10% by mole or more, 20% by mole or more, 30% by mole or more, 50% by mole or more, or 60% by mole or more, and 100% by mole or less, 99% by mole or less, 90% by mole or less, 50% by mole or less, or 40% by mole or less, specifically, 1% by mole to 100% by mole, 1% by mole to 99% by mole, or 30% by mole to 100% by mole, based on the number of moles of the total glycols.

Specific examples of the derivative of 1,4-cyclohexanedimethanol may include 4-(hydroxymethyl)cyclohexylmethyl-4-(hydroxymethyl)cyclohexanecarboxylate, 4-(4-(hydroxymethyl)cyclohexylmethoxymethyl)cyclohexylmethanol, or a mixture thereof. More specifically, it may be a mixture comprising 4-(hydroxymethyl)cyclohexylmethyl-4-(hydroxymethyl)cyclohexanecarboxylate and 4-(4-(hydroxymethyl)cyclohexylmethoxymethyl)cyclohexylmethanol at a molar ratio of 1:1 to 1:5 or 1:2 to 1:4.

The glycol monomer may have a molecular weight of, for example, less than 500, less than 400, less than 350, less than 300, or less than 250.

The polymer glycol may be, for example, selected from the group consisting of polyethylene glycol, polypropylene glycol, polytetramethylene glycol, polyhexamethylene glycol, copolymers of ethylene oxide and tetrahydrofuran, ethylene oxide-adducted polypropylene glycol, polycarbonatediol, polyneopentyl glycol, poly-3-methylpentanediol, and poly-1,5-pentanediol. More specifically, the polymer glycol may be at least one selected from the group consisting of polytetramethylene glycol, polycarbonatediol, polypropylene glycol, and ethylene oxide-adducted polypropylene glycol.

The polymer glycol may have a number average molecular weight of, for example, 400 or more, 500 or more, 600 or more, 700 or more, or 800 or more, and may be 6,000 or less, 5,000 or less, 4,000 or less, or 3,000 or less. As a specific example, the number average molecular weight of the polymer glycol may be 400 to 5,000. More specifically, it may preferably be 1,000 to 3,000 from the viewpoint of the reduction in phase separation.

The polymer glycol is used in an amount of 5% by weight to 75% by weight, specifically, 10% by weight to 60% by weight, more specifically, 15% by weight to 50% by weight, based on the weight of a final polyester resin, which is advantageous for achieving a high molecular weight while improving the elasticity of the polyester resin.

### Transesterification reaction

The glycol and bis(2-hydroxyethyl) terephthalate are subjected to a transesterification reaction.

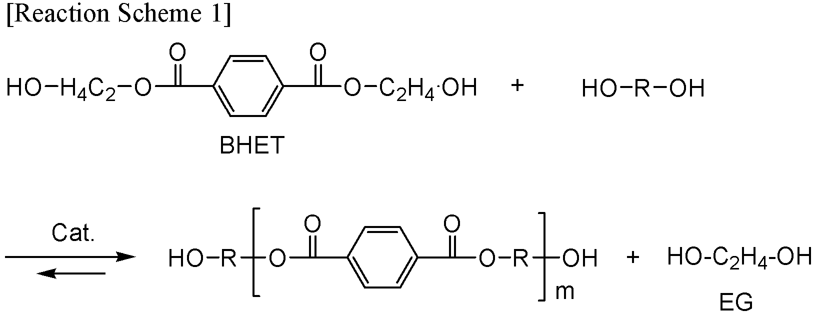

In Reaction Scheme 1, R is a group of a glycol having 3 or more carbon atoms from which the OH groups at both ends are excluded. As an example, R may be an alkylene group having 3 to 20 carbon atoms or a group in which two or more identical or different alkylene groups having 2 to 10 carbon atoms are connected via an ether group or a carbonate group. As another example, R may be a group of 3 to 20 carbon atoms containing single or multiple rings, wherein the ring may be aliphatic or aromatic and may also contain one or more heteroatoms (e.g., O, N, and S).

In Reaction Scheme 1, m is, for example, a number in the range of 2 to 10.

The transesterification reaction may be carried out in the presence of a catalyst. Thus, when the glycol, acid, or bis(2-hydroxyethyl) terephthalate is fed to the reactor, a catalyst may be fed together.

As the catalyst for the transesterification reaction, for example, at least one selected from the group consisting of a zinc-based catalyst, a titanium-based catalyst, a germanium-based catalyst, an antimony-based catalyst, an aluminum-based catalyst, and a tin-based catalyst may be used.

Examples of the zinc-based catalyst include zinc acetate, zinc acetate hydrate, zinc chloride, zinc sulfate, zinc sulfide, zinc carbonate, zinc citrate, zinc gluconate, or mixtures thereof. Examples of the titanium-based catalyst include tetraethyl titanate, acetyltripropyl titanate, tetrapropyl titanate, tetrabutyl titanate, 2-ethylhexyl titanate, octylene glycol titanate, triethanolamine titanate, acetylacetonate titanate, ethylacetoacetic ester titanate, isostearyl titanate, titanium dioxide, and the like. Examples of the germanium-based catalyst include germanium dioxide, germanium tetrachloride, germanium ethylene glycol oxide, germanium acetate, or combinations thereof. Specifically, germanium dioxide can be used as the germanium-based catalyst. Both crystalline and amorphous germanium dioxide may be used as the germanium dioxide, and glycol-soluble ones may also be used.

The amount of the transesterification catalyst employed may vary depending on the reaction conditions and the catalyst used. As an example, a metal-based catalyst (e.g., titanium-based catalyst, tin-based catalyst) may be employed in an amount of 0.0001 part by weight to 0.05 part by weight relative to 100 parts by weight of the glycol and bis(2-hydroxyethyl) terephthalate fed to a reactor.

The transesterification reaction may be carried out in a batch or continuous type.

As an example, when a glycol, or a glycol with an acid, is fed to a reactor, and the temperature reaches a certain level when the temperature is raised, bis(2-hydroxyethyl) terephthalate may be fed. The feeding of bis(2-hydroxyethyl) terephthalate may be carried out, for example, while ethylene glycol as a by-product is collected and removed in a nitrogen atmosphere at a temperature of 120°C to 280°C. As a specific example, the collection and removal of ethylene glycol may be carried out at a temperature of 120°C to 260°C and a pressure of 0.1 kgf/cm² to 2.0 kgf/cm². The ethylene glycol collected and removed may be purified and then supplied again for at least one of the transesterification reaction and the polymerization reaction.

The bis(2-hydroxyethyl) terephthalate may be fed all at once to be subjected to a transesterification reaction with a glycol. Alternatively, bis(2-hydroxyethyl) terephthalate may be introduced into the transesterification reaction with a glycol in a divided or continuous manner. In addition, the bis(2-hydroxyethyl) terephthalate may be fed to the transesterification reaction in the form of powder or solution. The solution of bis(2-hydroxyethyl) terephthalate may be prepared by mixing bis(2-hydroxyethyl) terephthalate with at least one solvent of water and ethylene glycol at 60°C to 190°C and adjusting the concentration to 50% by weight to 95% by weight.

According to an embodiment, bis(2-hydroxyethyl) terephthalate may be introduced into the transesterification reaction with a glycol in a divided manner of two or more times. The number of divided introductions may be 2 times or more, 3 times or more, 4 times or more, or 5 times or more, and may be 100 times or fewer, 50 times or fewer, 30 times or fewer, 20 times or fewer, 15 times or fewer, or 10 times or fewer. As a specific example, the number of divided introductions may be 2 to 30 times or 3 to 15 times. The time interval between the divided introductions may be determined by dividing the total introduction time by the number of divided introductions. The total introduction time may be, for example, 1 hour or longer or 2 hours or longer, and may be 5 hours or shorter or 4 hours or shorter. In addition, the amount of introduction at one time during the divided introductions may be determined by dividing the total amount of bis(2-hydroxyethyl) terephthalate to be introduced into the reaction by the number of divided introductions.

According to another embodiment, bis(2-hydroxyethyl) terephthalate is introduced into the transesterification reaction with a glycol in a continuous manner. The total time of continuous introduction may be, for example, 1 hour or longer or 2 hours or longer, and may be 5 hours or shorter or 4 hours or shorter. For example, the continuous introduction may be to introduce a constant amount of bis(2-hydroxyethyl) terephthalate per hour. The amount of introduction per hour may be determined by dividing the total amount of bis(2-hydroxyethyl) terephthalate to be introduced into the reaction by the total introduction time. As a specific example, bis(2-hydroxyethyl) terephthalate may be prepared in a powder form or in an aqueous BHET solution having a concentration of about 10 to 20% by weight as dissolved in water at about 80 to 100°C, which may be continuously fed. The continuous introduction may be carried out from the beginning of the transesterification reaction until one hour before the completion of the reaction. A dropping funnel may be used at the laboratory level, or a metered feeder may be used at the commercial level, for the continuous introduction of a constant amount per hour.

When the feeding of bis(2-hydroxyethyl) terephthalate is completed, the reaction conditions may be maintained until the transesterification reaction is completed. In addition, a process of collecting and removing ethylene glycol as a by-product may be continued during the transesterification reaction. The collection and removal of ethylene glycol may be carried out by a distillation process using a difference in boiling point from the other components. Ethylene glycol thus distilled off may be recovered by cooling and reused in other processes.

The termination point of the transesterification reaction may be determined by considering the theoretical amount of ethylene glycol formed from bis(2-hydroxyethyl) terephthalate through the transesterification reaction or as the point when the by-product is no longer released.

The pressure (absolute pressure) during the transesterification reaction may be, for example, 0.1 kgf/cm² or more, 0.2 kgf/cm² or more, or 0.5 kgf/cm² or more, and 2.5 kgf/cm² or less, 2.0 kgf/cm² or less, or 1.5 kgf/cm² or less, specifically, 0.5 kgf/cm² to 2.0 kgf/cm².

In addition, the temperature during the transesterification reaction may be 120°C or higher, 140°C or higher, 160°C or higher, 180°C or higher, or 200°C or higher, and may be 300°C or lower, 280°C or lower, 260°C or lower, 250°C or lower, or 220°C or lower.

As a specific example, the transesterification reaction may be carried out at a temperature of 120°C to 260°C and a pressure of 0.1 kgf/cm² to 2.0 kgf/cm².

In addition, the transesterification reaction may be carried out under a nitrogen atmosphere.

The pressure and temperature conditions during the transesterification reaction may be appropriately adjusted according to the specific characteristics of a polyester to be produced, the ratio of each component, or process conditions. For example, the transesterification reaction may be carried out at a temperature of 180°C or higher to smoothly collect and remove ethylene glycol, a by-product formed during the transesterification reaction. In addition, it may be carried out at a temperature lower than the boiling point of the glycol by 10°C in order to reduce the loss of the glycol for the substitution.

The product of the transesterification reaction mainly comprises a bis(glycol) terephthalate oligomer and derivatives thereof in which ethylene glycol residues in bis(2-hydroxyethyl) terephthalate are substituted with other glycol residues.

Accordingly, the present invention provides a process for preparing a bis(glycol) terephthalate oligomer, which comprises subjecting bis(2-hydroxyethyl) terephthalate to a transesterification reaction with a glycol having 3 or more carbon atoms; and collecting and removing ethylene glycol as a by-product formed during the transesterification reaction.

In addition, the present invention provides a bis(glycol) terephthalate oligomer, that is, a recycled bis(glycol) terephthalate oligomer, prepared by the above process.

According to an embodiment, the bis(glycol) terephthalate oligomer obtained by the transesterification reaction comprises a compound represented by the following Formula 1.

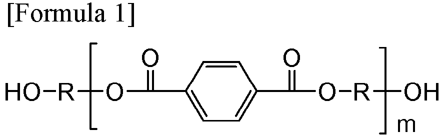

In Formula 1, -O-R-OH is a group derived from a glycol having 3 or more carbon atoms, and m is, for example, a number in the range of 2 to 10.

As described above, the bis(glycol) terephthalate oligomer may have 2 to 10 repeat units. For example, the number of repeat units in the bis(glycol) terephthalate oligomer may be 2 or more or 3 or more, and 10 or less, 8 or less, 6 or less, or 4or less.

In Formula 1, R is a group derived from a glycol having 3 or more carbon atoms. As an example, R may be an alkylene group having 3 to 20 carbon atoms or a group in which two or more identical or different alkylene groups having 2 to 10 carbon atoms are connected via an ether group or a carbonate group. As another example, R may be a group of 3 to 20 carbon atoms containing single or multiple rings, wherein the ring may be aliphatic or aromatic and may also contain one or more heteroatoms (e.g., O, N, and S).

As a specific example, Formula 1 may be a compound in which R is a group derived from diethylene glycol, that is, bis(diethylene glycol) terephthalate oligomer (hereinafter, abbreviated as BHDT oligomer). As another specific example, Formula 1 may be a compound in which R is a group derived from isosorbide, that is, bis(isosorbide) terephthalate oligomer (hereinafter, abbreviated as BHIT oligomer). As another specific example, Formula 1 may be a compound in which R is a group derived from 1,4-cyclohexanedimethanol, that is, bis(1,4-cyclohexanedimethanol) terephthalate oligomer (hereinafter, abbreviated as BHCT oligomer).

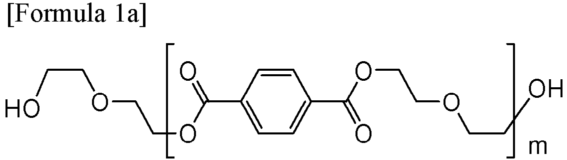

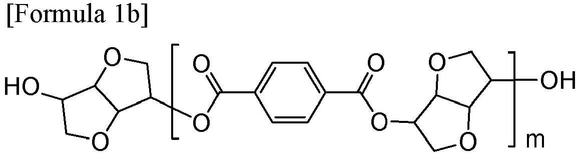

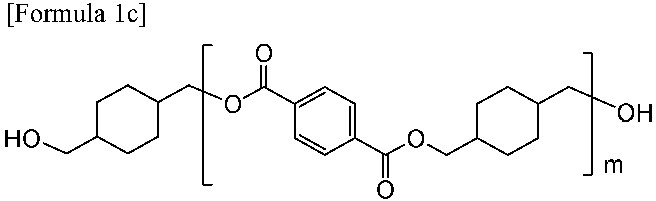

In Formulae 1a to 1c, m is each a number in the range of 2 to 10.

The bis(glycol)terephthalate oligomer may comprise two or more compounds represented by Formula 1 or derivatives thereof.

Since impurities in the bis(glycol) terephthalate oligomer are removed in the process of collecting and removing by-products such as a glycol formed during the transesterification reaction, its purity may be enhanced as compared with bis(2-hydroxyethyl) terephthalate as a starting material. For example, bis(glycol) terephthalate obtained through the transesterification reaction may have a purity of 80% or more. More specifically, the purity of the bis(glycol) terephthalate oligomer may be 85% or more, 90% or more, or 95% or more. As a specific example, it may be 80% to 99.9%.

### Preparation of a polyester resin

Thereafter, a polyester resin is prepared by conducting a polymerization using the bis(glycol) terephthalate oligomer.

In the polymerization step, an esterification reaction (first polymerization reaction step) and a polycondensation reaction (second polymerization reaction step) may be sequentially carried out.

The polymerization step may be a step of preparing a copolymer. For example, the polymerization step may comprise subjecting the bis(glycol) terephthalate oligomer and at least one comonomer to an esterification reaction; and subjecting the esterification reaction product to a polycondensation to obtain a copolymer.

Reaction Scheme 2 below schematically shows the copolymerization reaction using BHET as a comonomer during which a glycol may be formed as a by-product.

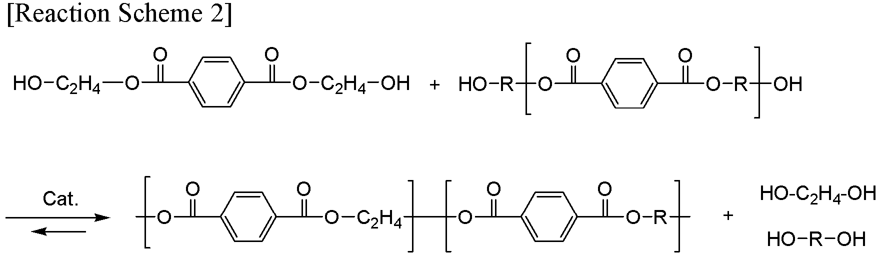

In Reaction Scheme 2, R is a group derived from a glycol having 3 or more carbon atoms.

According to the present invention, a step of collecting and removing ethylene glycol may be further carried out during at least one of the esterification reaction and the polycondensation reaction. The ethylene glycol collected and removed may be purified and then supplied again for at least one of the transesterification reaction and the polymerization step.

As described above, additional recycled monomers, in addition to the bis(glycol)terephthalate oligomer, may be added to the esterification reaction, and monomers other than the recycled raw materials may also be added. For example, at least one comonomer selected from the group consisting of bis(2-hydroxyethyl) terephthalate, a dicarboxylic acid, a dicarboxylic acid derivative, a diol, and a diol derivative may be further introduced into the esterification reaction.

As a specific example, the dicarboxylic acid may comprise terephthalic acid or isophthalic acid; the dicarboxylic acid derivative may comprise dimethyl terephthalate or dimethyl isophthalate; the glycol may comprise diethylene glycol, 1,4-cyclohexanedimethanol, isosorbide, 1,2-propanediol, 1,3-propanediol, 2-methyl-1,3-propanediol, 2-methylene-1,3-propanediol, 2-ethyl-1,3-propanediol, 2-isopropyl-1,3-propanediol, 2,2-dimethyl-1,3-propanediol, 1,4-butanediol, 2,3-butanediol, 3-methyl-1,5-pentanediol, 3-methyl-2,4-pentanediol, 1,6-hexanediol, 1,2-cyclohexanediol, or 1,4-cyclohexanediol; and the diol derivative may comprise 4-(hydroxymethyl)cyclohexylmethyl-4-(hydroxymethyl)cyclohexanecarboxylate or 4-(4-(hydroxymethyl)cyclohexylmethoxymethyl)cyclohexylmethanol. Such comonomers may be virgin monomers.

As another example, the at least one comonomer may comprise a recycled monomer obtained by the depolymerization of waste polyester. As a specific example, the at least one comonomer may comprise at least one recycled monomer selected from the group consisting of a recycled dicarboxylic acid, a recycled dicarboxylic acid derivative, a recycled diol, and a recycled diol derivative. As a more specific example, the recycled monomer may be selected from the group consisting of recycled ethylene glycol, recycled 1,4-cyclohexanedimethanol, recycled diethylene glycol, recycled isosorbide, recycled terephthalic acid, recycled dimethyl terephthalate, recycled isophthalic acid, and recycled dimethyl isophthalate. However, the type of the recycled monomer that can be used in the present invention as a comonomer is not limited thereto. Any recycled monomer that can be used in the preparation of a polyester resin can be used. Such a recycled monomer may be used in the polymerization of a polyester resin by completely replacing a virgin monomer. Alternatively, a recycled monomer and a virgin monomer may be mixed at a certain ratio and used in the polymerization of a polyester resin. The mixing ratio of the recycled monomer may be, for example, 0% by mole or more, 1% by mole or more, 10% by mole or more, 20% by mole or more, 30% by mole or more, 50% by mole or more, or 60% by mole or more, and 100% by mole or less, 99% by mole or less, 90% by mole or less, 50% by mole or less, or 40% by mole or less, specifically, 1% by mole to 100% by mole, 1% by mole to 99% by mole, or 30% by mole to 100% by mole, based on the number of moles of the total monomers.

The esterification reaction may be carried out in the presence of an esterification reaction catalyst. For example, a zinc-based compound may be used. Specific examples of the zinc-based catalyst include zinc acetate, zinc acetate hydrate, zinc chloride, zinc sulfate, zinc sulfide, zinc carbonate, zinc citrate, zinc gluconate, or mixtures thereof.

The esterification reaction may be carried out, for example, at a pressure of 0 kgf/cm² to 10.0 kgf/cm² and a temperature of 150°C to 300°C. The esterification reaction conditions may be appropriately adjusted according to the specific characteristics of the polyester to be produced, the ratio of each component, or process conditions. Specifically, the pressure in the esterification reaction may be 0 kg/cm² to 5.0 kg/cm², more specifically, 0.1 kg/cm² to 3.0 kg/cm². In addition, the temperature in the esterification reaction may be 200°C to 270°C, more specifically, 240°C to 260°C.

The esterification reaction may be carried out in a batch or continuous type. In addition, recycled BHET, a dicarboxylic acid, and a diol, which are raw materials, may be separately introduced into the reactor, or two or more raw materials may be introduced in a mixed state. They may be introduced in a solid, liquid, or slurry form. As an example, the dicarboxylic acid, the diol, and the recycled BHET may be added individually or in combination and may be mixed with a terephthalic acid oligomer prepared in advance. The terephthalic acid oligomer may be prepared by, for example, reacting terephthalic acid with a diol such as ethylene glycol, cyclohexanedimethanol, and isosorbide. As another example, they may be introduced in the form of a slurry in which a dicarboxylic acid and recycled BHET are mixed with a diol.

More specifically, a diol such as isosorbide, which is solid at room temperature, may be dissolved in water or ethylene glycol and then mixed with a dicarboxylic acid such as terephthalic acid to prepare a slurry. Alternatively, isosorbide may be melted at 60°C or higher, which is then mixed with a dicarboxylic acid such as terephthalic acid and other diols to prepare a slurry. In addition, water may be additionally added to the mixed slurry to help increase the fluidity of the slurry. In addition, in a continuous type, liquid raw materials (e.g., a solution of recycled BHET) may be continuously fed to a reactor using a pump or the like. The hourly feeding amount of raw materials can be determined by dividing the total amount of raw materials to be fed by time to achieve the target production per day.

The mixture of the bis(glycol) terephthalate oligomer, other monomers, and additive components is left in the esterification reactor for a certain period of time, for example, 1 hour to 24 hours or 4 hours to 10 hours, which is then transferred to a polycondensation reactor. The polycondensation reaction may produce a polyester resin having a relatively low molecular weight through melt polymerization. In addition, a polyester resin having a relatively high molecular weight may be produced through solid-state polymerization after the melt polymerization.

The temperature in the polycondensation reaction may be 150°C to 300°C, specifically, 200°C to 290°C, more specifically, 260°C to 280°C. In addition, the pressure in the polycondensation reaction may be 0.01 mmHg to 600 mmHg, specifically, 0.05 mmHg to 200 mmHg, more specifically, 0.1 mmHg to 100 mmHg. As the reduced pressure condition is adopted in the polycondensation reaction, a glycol, which is a by-product of the polycondensation reaction, can be collected and removed from the system. If the pressure in the polycondensation reaction exceeds the range of 0.01 mmHg to 400 mmHg, the removal of by-products may be insufficient. In addition, if the temperature in the polycondensation reaction is lower than 150°C, a glycol as a by-product of the reaction cannot be effectively collected and removed from the system; thus, the intrinsic viscosity of a final reaction product is low, resulting in a decrease in the physical properties of the final polyester resin. If the temperature in the polycondensation reaction exceeds 300°C, the possibility of yellowing of a final polyester resin increases. In addition, the polycondensation reaction may be carried out for a necessary period of time, for example, an average residence time of 1 hour to 24 hours, until the intrinsic viscosity of a final reaction product reaches an appropriate level.

In addition, the polycondensation reaction may be carried out in the presence of a polycondensation catalyst. The polycondensation catalyst may be, for example, a titanium-based compound, a germanium-based compound, an antimony-based compound, an aluminum-based compound, a tin-based compound, or a mixture thereof. Examples of the titanium-based compound include tetraethyl titanate, acetyltripropyl titanate, tetrapropyl titanate, tetrabutyl titanate, 2-ethylhexyl titanate, octylene glycol titanate, lactate titanate, triethanolamine titanate, acetylacetonate titanate, ethylacetoacetic ester titanate, isostearyl titanate, titanium dioxide, and the like. Examples of the germanium-based compound include germanium dioxide, germanium tetrachloride, germanium ethylene glycol oxide, germanium acetate, or mixtures thereof. Preferably, germanium dioxide can be used. Both crystalline and amorphous germanium dioxide may be used, and glycol-soluble ones may also be used. The amount of the polycondensation catalyst used may be such that the amount of titanium element relative to the weight of the polyester resin is about 1 ppm to 100 ppm, more preferably, about 1 ppm to 50 ppm.

In addition to the polycondensation catalyst, a stabilizer, a colorant, a crystallizing agent, an antioxidant, a branching agent, or the like may be further used. The timing of adding these additives is not particularly limited, and they may be added at any time during the preparation step of the polyester resin.

As the stabilizer, phosphorus-based compounds such as phosphoric acid, trimethyl phosphate, triethyl phosphate, and triethyl phosphonoacetate may be generally used. The amount thereof added may be such that 10 ppm to 200 ppm relative to the weight of the polyester resin based on the amount of elements. In addition, common colorants such as cobalt acetate and cobalt propionate may be exemplified as the colorant added to enhance the color of the polyester resin. The amount thereof added may be such that 10 ppm to 200 ppm relative to the weight of the polyester resin based on the amount of cobalt element. If necessary, anthraquinone-based compounds, perinone-based compounds, azo-based compounds, methine-based compounds, or the like may be used as an organic colorant. Commercially available toners such as Polysynthren Blue RLS from Clarient or Solvaperm Red BB from Clarient may be used. The amount of the organic compound colorant added may be adjusted to 0 to 50 ppm based on the weight of the polyester resin. A crystal nucleating agent, an ultraviolet absorber, a polyolefin resin, a polyamide resin, and the like may be exemplified as the crystallizing agent. Hindered phenol-based antioxidants, phosphite-based antioxidants, thioether-based antioxidants, or mixtures thereof may be exemplified as the antioxidant. Conventional branching agents having three or more functional groups, for example, trimellitic anhydride, trimethylol propane, trimellitic acid, or mixtures thereof may be exemplified as the branching agent.

### Polyester resin

According to another aspect of the present invention, there is provided a polyester resin prepared by the above process.

The polyester resin of the present invention is a polyester resin regenerated through the chemical recycling of waste polyester.

That is, the polyester resin is prepared by polymerizing raw materials that comprise a bis(glycol) terephthalate oligomer. In addition, the polyester resin may comprise bis(2-hydroxyethyl) terephthalate as a comonomer.

As described above, the bis(glycol) terephthalate oligomer may be a transesterification product between bis(2-hydroxyethyl) terephthalate and a glycol having 3 or more carbon atoms.

Here, the glycol has 3 or more carbon atoms, and the specific types thereof are as exemplified above.

As a specific example, the bis(glycol) terephthalate may be selected from the group consisting of bis(diethylene glycol) terephthalate, bis(isosorbide) terephthalate, bis(1,4-cyclohexanedimethanol) terephthalate, and derivatives thereof.

The total content of recycled raw materials in the polyester resin of the present invention may be 1% by weight or more, 5% by weight or more, 10% by weight or more, 25% by weight or more, 30% by weight or more, 50% by weight or more, 70% by weight or more, or 90% by weight or more. In addition, the total content of recycled raw materials may be 100% by weight or less, 99% by weight or less, 80% by weight or less, 60% by weight or less, 40% by weight or less, or 20% by weight or less. As a specific example, the polyester resin may comprise the bis(2-hydroxyethyl) terephthalate and bis(glycol) terephthalate oligomer in a total amount of 25% by weight or more based on the weight of the polyester resin.

Since monomers (i.e., virgin monomers) generally used in the polymerization of a polyester resin, for example, at least one selected from the group consisting of a dicarboxylic acid, a dicarboxylic acid derivative, a diol, and a diol derivative as described above, are further used in addition to recycled raw materials in the preparation of the polyester resin, the polyester resin may further comprise these comonomers.

In addition, the polyester resin may further comprise at least one recycled monomer obtained by the depolymerization of waste polyester. As a specific example, the at least one recycled monomer may be selected from the group consisting of a recycled dicarboxylic acid, a recycled dicarboxylic acid derivative, a recycled diol, and a recycled diol derivative. As a more specific example, the recycled monomer may be selected from the group consisting of recycled ethylene glycol, recycled 1,4-cyclohexanedimethanol, recycled diethylene glycol, recycled isosorbide, recycled terephthalic acid, recycled dimethyl terephthalate, recycled isophthalic acid, and recycled dimethyl isophthalate. However, the type of the recycled monomer that can be used in the present invention is not limited thereto. Any recycled monomer that can be used in the preparation of a polyester resin can be used. Such a recycled monomer may be contained in a polyester resin by completely replacing a virgin monomer. Alternatively, a recycled monomer and a virgin monomer may be together contained in a polyester resin at a certain ratio. The mixing ratio of the recycled monomer is as exemplified above in the process for preparing a polyester resin.

Since bis(2-hydroxyethyl) terephthalate used in the preparation of the polyester resin has a structure in which two ethylene glycols and one terephthalic acid are bonded, the polyester resin of the present invention may comprise a repeat unit derived from ethylene glycol and terephthalic acid. In addition, since the bis(glycol) terephthalate oligomer has a structure in which two ethylene glycols each having 3 or more carbon atoms and one terephthalic acid are bonded, the polyester resin of the present invention may further comprise a repeat unit derived from a glycol having 3 or more carbon atoms.

As described above, the polyester resin of the present invention comprises a dicarboxylic acid component and a glycol component as monomer components (polymer constituent unit) constituting the same. These may be derived from bis(2-hydroxyethyl) terephthalate and the glycol having 3 or more carbon atoms initially introduced for the preparation of the polyester resin, or a recycled bis(glycol) terephthalate oligomer prepared therefrom, and comonomers additionally introduced.

Accordingly, the polyester resin of the present invention may be a copolymerized resin comprising two or more dicarboxylic acid components and/or two or more diol components.

According to an embodiment, the diol component may further comprise a diol component, other than ethylene glycol, as a comonomer. The comonomer may comprise, for example, at least one selected from the group consisting of diethylene glycol, cyclohexanedimethanol, cyclohexanedimethanol derivatives, and isosorbide.

The diethylene glycol may contribute to enhancing the transparency and impact resistance of a polyester resin. For example, the diethylene glycol may be employed in an amount of 0.1% by mole to 50% by mole based on the number of moles of the entire diols.

The cyclohexanedimethanol (e.g., 1,2-cyclohexanedimethanol, 1,3-cyclohexanedimethanol, and 1,4-cyclohexanedimethanol) may contribute to enhancing the transparency and impact resistance of a polyester resin produced. For example, cyclohexanedimethanol may be employed in an amount of 5% by mole to 90% by mole based on the number of moles of the entire diol components. The cyclohexanedimethanol derivative may be 4-(hydroxymethyl)cyclohexylmethyl-4-(hydroxymethyl)cyclohexanecarboxylate or 4-(4-(hydroxymethyl)cyclohexylmethoxymethyl)cyclohexylmethanol. The cyclohexanedimethanol derivatives may be employed in an amount of 0.1% by mole to 25% by mole based on the number of moles of the entire diol components.

The isosorbide may enhance the processability of a final polyester resin. Although the transparency and impact resistance of a polyester resin are enhanced by the diol component of cyclohexanedimethanol and ethylene glycol, shear fluidization characteristics should be improved and the crystallization rate should be delayed for processability; however, it is difficult to achieve this effect with cyclohexanedimethanol and ethylene glycol alone. Thus, if isosorbide is employed as a diol component, the shear fluidization characteristics are improved and the crystallization rate is delayed while transparency and impact resistance are maintained, whereby it is possible to improve the processability of a polyester resin produced. Preferably, isosorbide may be employed in an amount of 0.1% by mole to 70% by mole based on the number of moles of the entire diol components.

The polyester resin may comprise terephthalic acid as a dicarboxylic acid component. For example, terephthalic acid may be employed in an amount of 5% by mole to 100% by mole based on the number of moles of the entire dicarboxylic acid. In addition, the terephthalic acid component may be formed from a terephthalic acid alkyl ester such as dimethyl terephthalic acid.

In addition, the dicarboxylic acid component may further comprise an aromatic dicarboxylic acid component, an aliphatic dicarboxylic acid component, or mixed components thereof, other than terephthalic acid. The dicarboxylic acid other than terephthalic acid may be employed in an amount of 1% by mole to 30% by mole based on the weight of the entire dicarboxylic acid components.

The aromatic dicarboxylic acid component may be an aromatic dicarboxylic acid having 8 to 20 carbon atoms, preferably, 8 to 14 carbon atoms, or a mixture thereof. Examples of the aromatic dicarboxylic acid include isophthalic acid, naphthalenedicarboxylic acids such as 2,6-naphthalenedicarboxylic acid, diphenyl dicarboxylic acid, 4,4'-stilbendicarboxylic acid, 2,5-furandicarboxylic acid, 2,5-thiophenedicarboxylic acid, and the like, but it is not limited thereto.

The aliphatic dicarboxylic acid component may be an aliphatic dicarboxylic acid having 4 to 20 carbon atoms, preferably, 4 to 12 carbon atoms, or a mixture thereof. Examples of the aliphatic dicarboxylic acid include linear, branched, or cyclic aliphatic dicarboxylic acid components such as cyclohexanedicarboxylic acids such as 1,4-cyclohexanedicarboxylic acid and 1,3-cyclohexanedicarboxylic acid, phthalic acid, sebacic acid, succinic acid, isodecylsuccinic acid, maleic acid, fumaric acid, adipic acid, glutaric acid, azelaic acid, and the like, but it is not limited thereto.

The polyester resin may also comprise a catalyst that has been used in the polymerization reaction for its preparation. For example, the polyester resin may comprise at least one catalyst selected from metal oxides and acetates. The metal contained in the catalyst may be selected from the group consisting of antimony (Sb), titanium (Ti), germanium (Ge), manganese (Mn), cobalt (Co), tin (Sn), and calcium (Ca).

The polyester resin may have a value obtained by subtracting the b value from the L value of 88 or more, 89 or more, 90 or more, 91 or more, 92 or more, or 93 or more, when Hunter Lab color space is measured. In addition, the upper limit of the L - b value is not particularly limited. But it may be, for example, 100 or less, 99 or less, 98 or less, 97 or less, or 95 or less. The measurement of the Hunter Lab color space may be carried out by making a specimen having a thickness of 6 mm with the polyester resin. As a specific example, the polyester resin may have a value obtained by subtracting the b value from the L value of 88 or more under the condition of a thickness of 6 mm when Hunter Lab color space is measured.

The intrinsic viscosity of the polyester resin at 35°C may be 0.5 dl/g or more, 0.6 dl/g or more, or 0.7 dl/g or more, and may be 1.2 dl/g or less, 1.1 dl/g or less, 1.0 dl/g or less, or 0.9 dl/g or less. For example, the polyester resin may have an intrinsic viscosity of 0.5 dl/g to 1.2 dl/g at 35°C.

The polyester resin according to the present invention can be used as a material for beverage-filling containers, packaging films, audio and video films, and the like by virtue of its excellent color, mechanical strength, thermal resistance, transparency, and gas barrier properties. In addition, polyester sheets or plates prepared from the polyester resin according to the present invention have good transparency and excellent mechanical strength, so that they can be used as raw materials for cases, boxes, partitions, shelves, panels, packaging materials, building materials, interior and exterior materials, and the like. In addition, the polyester resin according to the present invention can be used as an industrial material such as medical fibers and tire cords.

Accordingly, the present invention provides an article, which comprises the polyester resin. As an example, the article may be a film, a sheet, or a profile. Specific examples of the film include a heat shrinkable film and a blown film. The profile refers to a continuously extrusion-molded article of plastics excluding sheets and films. It may be manufactured by a general extrusion molding method and may have, for example, a tube or channel shape.

### Mode for the Invention

Hereinafter, a preferred embodiment is presented for the understanding of the present invention. However, the following examples are provided only to help easily understand the present invention, and the scope of the present invention is not limited thereby.

In the glycols used in the transesterification reaction and the dicarboxylic acids or diols used as comonomers in the polymerization of a polyester resin in the following examples, unless it is indicated as a recycled monomer (e.g., terephthalic acid, ethylene glycol, or the like), it should be understood that a virgin monomer was used.

### Preparation of recycled bis(2-hydroxyethyl) terephthalate

Recycled BHET of various purities was prepared by the depolymerization of a waste polyester resin by a known method or purchased commercially. Tables 1 and 2 below show the purity analyzed using HPLC for the recycled BHET used in each of the Preparation Examples or Examples.

### <Preparation of a bis(glycol) terephthalate oligomer through a transesterification reaction>

Various bis(glycol) terephthalate oligomers were each prepared by subjecting a glycol (CHDM, DEG, or ISB) and bis(2-hydroxyethyl) terephthalate to a transesterification reaction.

### Preparation Example: BHCT #1

A transesterification reactor equipped with a column and a condenser that can be cooled by water was prepared. The pressure in the reactor was adjusted to 2 kg/cm² by flowing nitrogen, and the temperature was raised while the pressure was maintained. When the temperature in the reactor reached about 250°C, while 1,4-cyclohexanedimethanol (CHDM, 511 kg/hr), bis(2-hydroxyethyl) terephthalate (BHET, 361 kg/hr), and zinc acetate (0.0178 kg/hr) were continuously fed, a transesterification reaction was carried out. Ethylene glycol, a by-product, was collected and removed through the column and condenser during the reaction. Even upon completion of the addition of raw materials, the transesterification reaction was continued for a total of 7 hours while maintaining 250°C until the discharge of ethylene glycol stopped. Upon completion of the transesterification reaction, nitrogen in the pressurized reactor was released to the outside to lower the pressure in the reactor to normal pressure to obtain a product. As a result, a total amount of 14.2 tons of a bis(1,4-cyclohexanedimethanol) terephthalate oligomer was obtained.

### Preparation Example: BHCT #2

The same procedure as in the Preparation Example BHCT #1 was repeated to obtain a total amount of 28.0 tons of a bis(1,4-cyclohexanedimethanol) terephthalate oligomer, except that recycled 1,4-cyclohexanedimethanol (r-CHDM, 1,208 kg/hr), bis(2-hydroxyethyl) terephthalate (BHET, 710 kg/hr), and zinc acetate (0.1168 kg/hr) were continuously fed to the reactor, and the transesterification reaction was carried out at a temperature of 150°C and a pressure of 0.1 kgf/cm² with ethylene glycol as a by-product collected and removed.

### Preparation Example: BHCT #3

The same procedure as in the Preparation Example BHCT #1 was repeated to obtain a total amount of 7.0 tons of a bis(1,4-cyclohexanedimethanol) terephthalate oligomer, except that 1,4-cyclohexanedimethanol (CHDM, 400 kg/hr), bis(2-hydroxyethyl) terephthalate (BHET, 176 kg/hr), and zinc acetate (0.0145 kg/hr) were continuously fed to the reactor, and the transesterification reaction was carried out for 2 hours at a temperature of 220°C and a pressure of 1 kgf/cm² with ethylene glycol as a by-product collected and removed.

### Preparation Example: BHCT #4

The same procedure as in the Preparation Example BHCT #1 was repeated to obtain a total amount of 2.7 tons of a bis(1,4-cyclohexanedimethanol) terephthalate oligomer, except that 1,4-cyclohexanedimethanol (CHDM, 191 kg/hr), bis(2-hydroxyethyl) terephthalate (BHET, 67 kg/hr), and zinc acetate (0.0011 kg/hr) were continuously fed to the reactor, and the transesterification reaction was carried out for 6 hours at a temperature of 190°C and a pressure of 1 kgf/cm² with ethylene glycol as a by-product collected and removed.

### Preparation Example: BHDT #1

The same procedure as in the Preparation Example BHCT #1 was repeated to obtain a total amount of 2.2 tons of a bis(diethylene glycol) terephthalate oligomer, except that diethylene glycol (DEG, 42 kg/hr), bis(2-hydroxyethyl) terephthalate (BHET, 67 kg/hr), and zinc acetate (0.0063 kg/hr) were continuously fed to the reactor, and the transesterification reaction was carried out for 6 hours at a temperature of 190°C and a pressure of 1 kgf/cm² with ethylene glycol as a by-product collected and removed.

### Preparation Example: BHDT #2

The same procedure as in the Preparation Example BHCT #1 was repeated to obtain a total amount of 4.5 tons of a bis(diethylene glycol) terephthalate oligomer, except that recycled diethylene glycol (r-DEG, 173 kg/hr), bis(2-hydroxyethyl) terephthalate (BHET, 139 kg/hr), and zinc acetate (0.0112 kg/hr) were continuously fed to the reactor, and the transesterification reaction was carried out for 4 hours at a temperature of 170°C and a pressure of 2 kgf/cm² with ethylene glycol as a by-product collected and removed.

### Preparation Example: BHIT #1

The same procedure as in the Preparation Example BHCT #1 was repeated to obtain a total amount of 7.8 tons of a bis(isosorbide) terephthalate oligomer, except that isosorbide (ISB, 344 kg/hr), bis(2-hydroxyethyl) terephthalate (BHET, 200 kg/hr), and zinc acetate (0.0033 kg/hr) were continuously fed to the reactor, and the transesterification reaction was carried out for 2 hours at a temperature of 160°C and a pressure of 0.5 kgf/cm² with ethylene glycol as a by-product collected and removed.

### Preparation Example: BHIT #2

The same procedure as in the Preparation Example BHCT #1 was repeated to obtain a total amount of 7.5 tons of a bis(isosorbide) terephthalate oligomer, except that recycled isosorbide (r-ISB, 440 kg/hr), bis(2-hydroxyethyl) terephthalate (BHET, 192 kg/hr), and zinc acetate (0.0156 kg/hr) were continuously fed to the reactor, and the transesterification reaction was carried out for 2 hours at a temperature of 190°C and a pressure of 0.5 kgf/cm² with ethylene glycol as a by-product collected and removed.

The processes of the Preparation Examples are summarized in Table 1 below. In addition, the molecular weight of each bis(glycol) terephthalate obtained in the Preparation Examples was measured by gel permeation chromatography (GPC). The number of repeat units (units in which terephthalic acid and diol were condensed) was calculated therefrom and shown in the table below.

**[Table 1]**

| | | BHCT #1 | BHCT #2 | BHCT #3 | BHCT #4 | BHDT #1 | BHDT #2 | BHIT #1 | BHIT #2 |
|---|---|---|---|---|---|---|---|---|---|
| Purity of recycled BHET (%) | | 95 | 80 | 85 | 90 | 65 | 85 | 80 | 80 |
| Feeding | BHET (mole) | 100 | 100 | 100 | 100 | 100 | 100 | 100 | 100 |
| | CHDM (mole) | 250 | - | 400 | 500 | - | - | - | - |
| | r-CHDM (mole) | - | 300 | - | - | - | - | - | - |
| | DEG (mole) | - | - | - | - | 150 | - | - | - |
| | r-DEG (mole) | - | - | - | - | - | 300 | - | - |
| | ISB (mole) | - | - | - | - | - | - | 300 | - |
| | r-ISB (mole) | - | - | - | - | - | - | - | 400 |
| | Catalyst (ppm) | 30 | 100 | 50 | 10 | 70 | 60 | 10 | 50 |
| Reaction | Temp. (°C) | 250 | 150 | 220 | 190 | 190 | 170 | 160 | 190 |
| | Pressure (kgf/cm²) | 2 | 0.1 | 1 | 1 | 1 | 2 | 0.5 | 0.5 |
| | Time (hr) | 7 | 5 | 2 | 6 | 6 | 4 | 2 | 2 |
| Analysis | No. of repeat units | 10 | 8 | 4 | 3 | 3.5 | 2 | 2 | 3 |
| * The amount (mole) of each monomer added refers to the relative molar ratio. | | | | | | | | | |

### <Preparation of a polyester resin>

A polyester resin was prepared using the bis(glycol) terephthalate prepared above.

### Example 1

Each bis(glycol) terephthalate oligomer obtained in the Preparation Examples was transferred to an esterification reactor, and the other polymerization raw materials and catalyst were added. Nitrogen was injected into the reactor to make the reactor pressurized by 0.5 kgf/cm² higher than normal pressure. The temperature of the esterification reactor was raised to 260°C, and bis(diethylene glycol) terephthalate oligomer (BHDT #1, 90.5 kg/hr), bis(2-hydroxyethyl) terephthalate (BHET, 1,345.0 kg/hr), terephthalic acid (TPA, 835.0 kg/hr), ethylene glycol (EG, 72.2 kg/hr), 1,4-cyclohexanedimethanol (CHDM, 30.5 kg/hr), diethylene glycol (DEG, 22.5 kg/hr), a Ge catalyst (1.3 kg/hr), a Ti catalyst (0.19 kg/hr), phosphoric acid (0.208 kg/hr), a blue toner (0.008 kg/hr), and a red toner (0.002 kg/hr) were continuously fed to the esterification reactor. An esterification reaction was carried out at 260°C for about 7 hours, the product was then transferred to a polycondensation reactor, and by-products formed during the reaction were collected and removed through a column and a condenser.

Then, the pressure of the polycondensation reactor was reduced from normal pressure to 5 Torr (absolute pressure: 5 mmHg) over 30 minutes. At the same time, the temperature of the polycondensation reactor was raised to 275°C over 1 hour, and a polycondensation reaction was then carried out while the pressure of the polycondensation reactor was maintained at 1 Torr (absolute pressure: 1 mmHg) or less. At the beginning of the polycondensation reaction, the stirring speed was set high. As the polycondensation reaction proceeded, when the stirring power was weakened due to the increase in the viscosity of the reactants or the temperature of the reactants rose above the set temperature, the stirring speed was appropriately adjusted accordingly. The polycondensation reaction was carried out for 7 hours until the intrinsic viscosity (IV) of the mixture (melt) in the reactor reached 0.70 dl/g. Thereafter, the mixture was discharged to the outside of the reactor to form pellets, which were solidified with a cooling liquid and then granulated to have an average weight of about 12 to 14 mg.

The granules were left at 150°C for 1 hour to be crystallized, which were then fed to a solid-state polymerization reactor. While nitrogen flowed at a rate of 50 L/minute, the temperature of the reactor was raised from room temperature to 200°C at a rate of 40°C/hour. While it was maintained, a solid-state polymerization was carried out until the intrinsic viscosity (IV) of the granules in the reactor reached 0.90 dl/g to obtain about 50 tons of a polyester resin (copolymer).

### Example 2

The same procedure as in Example 1 was repeated to obtain about 50 tons of a polyester resin (copolymer), except that the bis(1,4-cyclohexanedimethanol) terephthalate oligomer (BHCT #1,592.9 kg/hr), bis(2-hydroxyethyl) terephthalate (BHET, 1,801.4 kg/hr), terephthalic acid (TPA, 157.0 kg/hr), 1,4-cyclohexanedimethanol (CHDM, 54.5 kg/hr), diethylene glycol (DEG, 22.5 kg/hr), a CHDM derivative (comprising 4-(hydroxymethyl)cyclohexylmethyl-4-(hydroxymethyl)cyclohexanecarboxylate and 4-(4-(hydroxymethyl)cyclohexylmethoxymethyl)cyclohexylmethanol at a molar ratio of 1:3, 41.4 kg/hr), a Ge catalyst (2.7 kg/hr), a Ti catalyst (0.19 kg/hr), phosphoric acid (0.208 kg/hr), a blue toner (0.005 kg/hr), and a red toner (0.001 kg/hr) were continuously fed, the esterification reaction was carried out at a pressure higher than normal pressure by 2.0 kgf/cm² and a temperature of 255°C for 5 hours, the polycondensation reaction was carried out at a temperature of 285°C until the intrinsic viscosity (IV) reached 0.78 dl/g for 10 hours, and no solid-state polymerization was carried out.

### Example 3

The same procedure as in Example 1 was repeated to obtain about 50 tons of a polyester resin (copolymer), except that the bis(isosorbide) terephthalate oligomer (BHIT #1, 325.2 kg/hr), the bis(1,4-cyclohexanedimethanol) terephthalate oligomer (BHCT #2, 1,167.5 kg/hr), bis(2-hydroxyethyl) terephthalate (BHET, 554.3 kg/hr), terephthalic acid (TPA, 492.6 kg/hr), 1,4-cyclohexanedimethanol (CHDM, 75.4 kg/hr), isosorbide (ISB, 76.5 kg/hr), diethylene glycol (DEG, 9.3 kg/hr), a Ge catalyst (13.3 kg/hr), phosphoric acid (0.042 kg/hr), a blue toner (0.006 kg/hr), and a red toner (0.002 kg/hr) were continuously fed, the esterification reaction was carried out at a pressure higher than normal pressure by 1.0 kgf/cm² and a temperature of 265°C for 4 hours, the polycondensation reaction was carried out at a temperature of 275°C until the intrinsic viscosity (IV) reached 0.70 dl/g for 6 hours, and no solid-state polymerization was carried out.

### Example 4

The same procedure as in Example 1 was repeated to obtain about 50 tons of a polyester resin (copolymer), except that the bis(diethylene glycol) terephthalate oligomer (BHDT #2, 186.4 kg/hr), the bis(1,4-cyclohexanedimethanol) terephthalate oligomer (BHCT #3, 290.0 kg/hr), bis(2-hydroxyethyl) terephthalate (BHET, 251.8 kg/hr), terephthalic acid (TPA, 1,275.2 kg/hr), ethylene glycol (EG, 347.2 kg/hr), 1,4-cyclohexanedimethanol (CHDM, 28.5 kg/hr), diethylene glycol (DEG, 5.3 kg/hr), a CHDM derivative (comprising 4-(hydroxymethyl)cyclohexylmethyl-4-(hydroxymethyl)cyclohexanecarboxylate and 4-(4-(hydroxymethyl)cyclohexylmethoxymethyl)cyclohexylmethanol at a molar ratio of 1:3, 86.8 kg/hr), a Ge catalyst (1.3 kg/hr), phosphoric acid (0.208 kg/hr), a blue toner (0.010 kg/hr), and a red toner (0.004 kg/hr) were continuously fed, the esterification reaction was carried out at a pressure higher than normal pressure by 0.5 kgf/cm² and a temperature of 260°C for 8 hours, the polycondensation reaction was carried out at a temperature of 275°C until the intrinsic viscosity (IV) reached 0.75 dl/g for 9 hours, and no solid-state polymerization was carried out.

### Example 5

The same procedure as in Example 1 was repeated to obtain about 50 tons of a polyester resin (copolymer), except that the bis(1,4-cyclohexanedimethanol) terephthalate oligomer (BHCT #4, 110.9 kg/hr), bis(2-hydroxyethyl) terephthalate (BHET, 1,886.4 kg/hr), terephthalic acid (TPA, 484.3 kg/hr), isophthalic acid (IPA, 1,276.9 kg/hr), ethylene glycol (EG, 11.0 kg/hr), isosorbide (ISB, 20.7 kg/hr), diethylene glycol (DEG, 22.5 kg/hr), a Ti catalyst (0.19 kg/hr), phosphoric acid (4.167 kg/hr), cobalt acetate (0.4 kg/hr), a blue toner (0.010 kg/hr), and a red toner (0.004 kg/hr) were continuously fed, the esterification reaction was carried out at a pressure higher than normal pressure by 3.0 kgf/cm² and a temperature of 260°C for 5 hours, the polycondensation reaction was carried out at a temperature of 280°C until the intrinsic viscosity (IV) reached 0.60 dl/g for 4 hours, and the solid-state polymerization was carried out until the intrinsic viscosity (IV) reached 1.10 dl/g.

### Example 6

The same procedure as in Example 1 was repeated to obtain about 50 tons of a polyester resin (copolymer), except that the bis(isosorbide) terephthalate oligomer (BHIT #2, 312.2 kg/hr), bis(2-hydroxyethyl) terephthalate (BHET, 765.8 kg/hr), terephthalic acid (TPA, 1,042.7 kg/hr), ethylene glycol (EG, 249.2 kg/hr), 1,4-cyclohexanedimethanol (CHDM, 434.2 kg/hr), isosorbide (ISB, 73.4 kg/hr), a Ti catalyst (0.19 kg/hr), phosphoric acid (2.083 kg/hr), cobalt acetate (0.1 kg/hr), a blue toner (0.004 kg/hr), and a red toner (0.002 kg/hr) were continuously fed, the esterification reaction was carried out at a pressure higher than normal pressure by 3.0 kgf/cm² and a temperature of 260°C for 5 hours, the polycondensation reaction was carried out at a temperature of 280°C until the intrinsic viscosity (IV) reached 0.55 dl/g for 3 hours, and no solid-state polymerization was carried out.

### Comparative Example 1

The same procedure as in Example 1 was repeated to obtain about 50 tons of a polyester resin (copolymer), except that bis(2-hydroxyethyl) terephthalate (BHET, 1,441.1 kg/hr), ethylene glycol (EG, 10.6 kg/hr), 1,4-cyclohexanedimethanol (CHDM, 277.8 kg/hr), a CHDM derivative (comprising 4-(hydroxymethyl)cyclohexylmethyl-4-(hydroxymethyl)cyclohexanecarboxylate and 4-(4-(hydroxymethyl)cyclohexylmethoxymethyl)cyclohexylmethanol at a molar ratio of 1:3, 24.9 kg/hr), a Ge catalyst (1.6 kg/hr), a Ti catalyst (0.11 kg/hr), phosphoric acid (0.125 kg/hr), a blue toner (0.003 kg/hr), and a red toner (0.001 kg/hr) were continuously fed, the esterification reaction was carried out at a pressure higher than normal pressure by 2.0 kgf/cm² and a temperature of 255°C for 7 hours, the polycondensation reaction was carried out at a temperature of 285°C until the intrinsic viscosity (IV) reached 0.78 dl/g for 12 hours, and no solid-state polymerization was carried out.

### Comparative Example 2

The same procedure as in Example 1 was repeated to obtain about 50 tons of a polyester resin (copolymer), except that bis(2-hydroxyethyl) terephthalate (BHET, 335.1 kg/hr), terephthalic acid (TPA, 657.0 kg/hr), ethylene glycol (EG, 26.2 kg/hr), 1,4-cyclohexanedimethanol (CHDM, 493.9 kg/hr), a CHDM derivative (comprising 4-(hydroxymethyl)cyclohexylmethyl-4-(hydroxymethyl)cyclohexanecarboxylate and 4-(4-(hydroxymethyl)cyclohexylmethoxymethyl)cyclohexylmethanol at a molar ratio of 1:3, 24.9 kg/hr), a Ge catalyst (8.0 kg/hr), phosphoric acid (0.025 kg/hr), a blue toner (0.004 kg/hr), and a red toner (0.001 kg/hr) were continuously fed, the esterification reaction was carried out at a pressure higher than normal pressure by 1.0 kgf/cm² and a temperature of 265°C for 5 hours, the polycondensation reaction was carried out at a temperature of 275°C until the intrinsic viscosity (IV) reached 0.70 dl/g for 5 hours, and no solid-state polymerization was carried out.

The raw materials employed in the Examples and Comparative Examples are summarized in Table 2 below.

**[Table 2]**

| | | Ex. 1 | Ex. 2 | Ex. 3 | Ex. 4 | Ex. 5 | Ex. 6 | C. Ex. 1 | C. Ex. 2 |
|---|---|---|---|---|---|---|---|---|---|
| Purity of recycled BHET (%) | | 65 | 95 | 80 | 85 | 90 | 90 | 90 | 77 |
| | BHET | 50.0 | 75.0 | 25.0 | 10.0 | 70.0 | 30.0 | 100.0 | 25.0 |
| | BHDT | 2.5 | - | - | 5.5 | - | - | - | - |
| | BHIT | - | - | 9.0 | - | - | 7.5 | - | - |
| | BHCT | - | 15.0 | 32.0 | 7.0 | 2.5 | - | - | - |
| Recycled raw material (mole) | r-TPA | - | - | 34.0 | 77.5 | - | - | - | 75.0 |
| | r-IPA | - | - | - | - | 27.5 | - | - | - |
| | r-DEG | 2.0 | 1.0 | - | - | - | - | - | - |
| | r-ISB | - | - | - | - | - | 5.0 | - | - |
| | r-CHDM | 2.0 | 4.0 | - | - | - | 30.0 | - | 65.0 |
| | r-EG | 11.0 | - | - | - | - | 40.0 | 3.0 | 8.0 |
| Virgin monomer (mole) | TPA | 47.5 | 10.0 | - | - | 27.5 | 62.5 | - | - |
| | IPA | - | - | - | - | - | - | - | - |
| | DEG | - | - | 1.0 | 0.5 | 2.0 | - | - | 2.0 |
| | ISB | - | - | 6.0 | - | 1.3 | - | - | 20.0 |
| | CHDM | - | - | 6.0 | 2.0 | - | - | 34.0 | - |
| | EG | - | - | - | 56.5 | 1.7 | - | - | - |
| | CHDM derivative | - | 3.0 | - | 6.0 | - | - | 3.0 | - |
| * The amount (mole) of each recycled monomer and virgin monomer added refers to the relative molar ratio. | | | | | | | | | |

### Test Example

The raw materials used and final resins in the Examples and Comparative Examples were tested as follows.

### (1) Amount of recycled BHET added

Moles of bis(2-hydroxyethyl) terephthalate (BHET) added based on the total number of moles of dicarboxylic acid and its derivatives (TPA, BHET, BHDT, BHIT, and BHCT) added for the preparation of a polyester resin was calculated in percentage (% by mole).

### (2) Content of the recycled raw materials in the resin

The total weight of the recycled monomers and oligomers (BHET, BHDT, BHIT, and BHCT) in percentage (% by weight) was calculated based on the total weight of the monomers and oligomers employed for the preparation of the polyester resin (BHET, BHDT, BHIT, BHCT, TPA, IPA, EG, CHDM, ISB, DEG, and the like).

### (3) Analysis of glycol recovery (by-products relative to production)

The total amount of by-products (glycols) collected and removed during the polymerization reaction (esterification reaction and polycondensation reaction) of the polyester resin was measured. The total amount (kg) of the by-products (glycols) collected and removed was divided by the total time (hr) required for the polymerization reaction (esterification reaction and polycondensation reaction) of the polyester resin to calculate the by-products relative to production (kg/hr).

The test results are shown in Table 3 below.

**[Table 3]**

| | Ex. 1 | Ex. 2 | Ex. 3 | Ex. 4 | Ex. 5 | Ex. 6 | C. Ex. 1 | C. Ex. 2 |
|---|---|---|---|---|---|---|---|---|
| Amount of recycled BHET employed | 50 | 75 | 25 | 10 | 70 | 30 | 100 | 25 |
| Recycled raw materials in the resin (% by weight) | 60 | 90 | 76 | 29 | 52 | 31 | 82 | 20 |
| By-products relative to production | 349 | 1,550 | 1,156 | 61 | 1,345 | 1,448 | 3,311 | 1,960 |

As can be seen from Table 3 above, the amount of glycols required to be collected and removed was significantly increased in Comparative Examples 1 and 2 as compared with Examples 1 to 6. In addition, the total content of recycled raw materials in the resin was low in Comparative Example 1 even though a larger amount of BHET than that of Example 2 was employed. The total content of recycled raw materials in the resin was lower in Comparative Example 2 even though the same amount of BHET as that of Example 2 was employed.

## Claims

1. A process for preparing a polyester resin, which comprises:
subjecting bis(2-hydroxyethyl) terephthalate to a transesterification reaction with a glycol having 3 or more carbon atoms;
collecting and removing ethylene glycol as a by-product formed during the transesterification reaction;
obtaining a bis(glycol) terephthalate oligomer as a product of the transesterification reaction; and
conducting a polymerization using the bis(glycol) terephthalate oligomer.

2. The process for preparing a polyester resin of claim 1, wherein the bis(2-hydroxyethyl) terephthalate is obtained by the depolymerization of waste polyester.

3. The process for preparing a polyester resin of claim 1, wherein the bis(2-hydroxyethyl) terephthalate has a purity of 60% to 97%.

4. The process for preparing a polyester resin of claim 1, wherein the glycol having 3 or more carbon atoms comprises at least one selected from the group consisting of 1,3-propanediol, 1,4-butanediol, 1,4-cyclohexanedimethanol, isosorbide, 2-methyl-1,3-propanediol, 2-methylene-1,3-propanediol, 2-ethyl-1,3-propanediol, 2-isopropyl-1,3-propanediol, 2,2-dimethyl-1,3-propanediol, 2,3-butanediol, 3-methyl-1,5-pentanediol, 3-methyl-2,4-pentanediol, 1,6-hexanediol, 1,2-cyclohexanediol, 1,4-cyclohexanediol, diethylene glycol, polyethylene glycol, polypropylene glycol, polytetramethylene glycol, polyhexamethylene glycol, copolymers of ethylene oxide and tetrahydrofuran, ethylene oxide-adducted polypropylene glycol, polycarbonate diol, polyneopentyl glycol, poly-3-methylpentanediol, poly-1,5-pentanediol, and derivatives thereof.

5. The process for preparing a polyester resin of claim 1, wherein the glycol having 3 or more carbon atoms comprises at least one type of recycled glycol obtained by the depolymerization of waste polyester.

6. The process for preparing a polyester resin of claim 1, wherein the bis(glycol) terephthalate oligomer has 2 to 10 repeat units.

7. The process for preparing a polyester resin of claim 1, wherein the polymerization step comprises:
subjecting the bis(glycol) terephthalate oligomer and at least one comonomer to an esterification reaction; and
subjecting the esterification reaction product to a polycondensation reaction to obtain a copolymer.

8. The process for preparing a polyester resin of claim 7, wherein at least one comonomer selected from the group consisting of a dicarboxylic acid, a dicarboxylic acid derivative, a diol, and a diol derivative is further introduced into the esterification reaction.

9. The process for preparing a polyester resin of claim 8, wherein the dicarboxylic acid comprises terephthalic acid or isophthalic acid;
the dicarboxylic acid derivative comprises dimethyl terephthalate or dimethyl isophthalate;
the glycol comprises diethylene glycol, 1,4-cyclohexanedimethanol, isosorbide, 1,2-propanediol, 1,3-propanediol, 2-methyl-1,3-propanediol, 2-methylene-1,3-propanediol, 2-ethyl-1,3-propanediol, 2-isopropyl-1,3-propanediol, 2,2-dimethyl-1,3-propanediol, 1,4-butanediol, 2,3-butanediol, 3-methyl-1,5-pentanediol, 3-methyl-2,4-pentanediol, 1,6-hexanediol, 1,2-cyclohexanediol, or 1,4-cyclohexanediol; and
the diol derivative comprises 4-(hydroxymethyl)cyclohexylmethyl-4-(hydroxymethyl)cyclohexanecarboxylate or 4-(4-(hydroxymethyl)cyclohexylmethoxymethyl)cyclohexylmethanol.

10. The process for preparing a polyester resin of claim 8, wherein the at least one comonomer comprises a recycled monomer obtained by the depolymerization of waste polyester.

11. The process for preparing a polyester resin of claim 7, which further comprises collecting and removing ethylene glycol during at least one of the esterification reaction and the polycondensation reaction.

12. The process for preparing a polyester resin of claim 1, wherein the ethylene glycol collected and removed is purified and then supplied again for at least one of the transesterification reaction and the polymerization step.

13. The process for preparing a polyester resin of claim 1, wherein the polyester resin comprises the bis(2-hydroxyethyl) terephthalate and the bis(glycol) terephthalate in a total amount of 25% by weight or more based on the weight of the polyester resin.

14. The process for preparing a polyester resin of claim 13, wherein the polyester resin further comprises at least one recycled monomer obtained by the depolymerization of waste polyester.

15. A process for preparing a bis(glycol) terephthalate oligomer, which comprises:
subjecting bis(2-hydroxyethyl) terephthalate to a transesterification reaction with a glycol having 3 or more carbon atoms; and
collecting and removing ethylene glycol as a by-product formed during the transesterification reaction.

16. The process for preparing a bis(glycol) terephthalate oligomer of claim 15, wherein the bis(2-hydroxyethyl) terephthalate is fed to the transesterification reaction in the form of powder or solution.

17. The process for preparing a bis(glycol) terephthalate oligomer of claim 15, wherein the solution of bis(2-hydroxyethyl) terephthalate is prepared by mixing the bis(2-hydroxyethyl) terephthalate with at least one solvent of water and ethylene glycol at 60°C to 190°C and adjusting the concentration to 50% by weight to 95% by weight.

18. The process for preparing a bis(glycol) terephthalate oligomer of claim 15, wherein the transesterification reaction and the collection and removal of ethylene glycol are carried out at a temperature of 120°C to 260°C and a pressure of 0.1 kgf/cm² to 2.0 kgf/cm².

19. A polyester resin, which is prepared by the process of claim 1.

20. A bis(glycol) terephthalate oligomer, which is prepared by the process of claim 15.
